(19) Europäisches Patentamt
European Patent Office
Office européen des brevets

(11) Veröffentlichungsnummer: **0 391 022 A2**

(12) # EUROPÄISCHE PATENTANMELDUNG

(21) Anmeldenummer: **90100891.2**

(22) Anmeldetag: **17.01.90**

(51) Int. Cl.[5]: **C07K 7/10, C12N 15/62, C12N 15/70**

Patentansprüche für folgende Vertragsstaaten: ES + GR

(30) Priorität: **21.01.89 DE 3901681**

(43) Veröffentlichungstag der Anmeldung: **10.10.90 Patentblatt 90/41**

(84) Benannte Vertragsstaaten: **AT BE CH DE DK ES FR GB GR IT LI LU NL SE**

(71) Anmelder: **BEHRINGWERKE Aktiengesellschaft**
**Postfach 1140**
**D-3550 Marburg 1(DE)**

(72) Erfinder: **Knapp, Stefan, Dr.**
**Zeppelinstrasse 33**
**D-3550 Marburg(DE)**
Erfinder: **Amann, Egon, Dr.**
**Sachsenring 8**
**D-3550 Marburg(DE)**
Erfinder: **Abel, Karl-Josef, Dr.**
**Am Ziegenberg 8**
**D-3550 Marburg(DE)**

(74) Vertreter: **Klein, Otto, Dr. et al**
**Hoechst AG Zentrale Patentabteilung**
**Postfach 80 03 20**
**D-6230 Frankfurt am Main 80(DE)**

(54) **Signalpeptid für die Sekretion von Peptiden in Escherichia coli, Verfahren zu seiner Gewinnung und seine Verwendung.**

(57) Die Erfindung betrifft ein neues Signalpeptid aus Bordetella pertussis mit der Aminosäuresequenz M K K W F V A A G I G A G L L M L S S A A sowie besonders geeignete Expressionsvektoren, mit deren Hilfe solche Signalsequenzen gefunden und/oder bewertet werden können.

EP 0 391 022 A2

# Signalpeptid für die Sekretion von Peptiden in Escherichia coli, Verfahren zu seiner Gewinnung und seine Verwendung

Die Erfindung betrifft das Signalpeptid eines Proteins aus Bordetella pertussis, welches in der Lage ist, heterologe Proteine in den periplasmatischen Raum zwischen innerer und äußerer Membran gramnegativer Bakterienspezies zu dirigieren. Die Erfindung betrifft weiterhin DNA-Sequenzen, die für dieses Signalpeptid kodieren, Plasmide, die eine solche Genstruktur enthalten, sowie Wirtsorganismen mit solchen Plasmiden. Weiter betrifft die Erfindung Plasmidvektoren, mit deren Hilfe die Effizienz bekannter und neuer Signalsequenzen bestimmt und verglichen werden kann. Als Folge solcher vergleichender Studien lassen sich besonders effiziente Signalsequenzen identifizieren, klonieren und in allen drei möglichen Translationsleserahmen für die Expression heterologer Proteine verwenden.

Grundsätzlich lassen sich zwei verschiedene Typen von Signalsequenzen unterscheiden: ein "hydrophober" Typ und ein "hydrophiler" Typ. Die "hydrophobe" Gruppe von Signalsequenzen umfaßt in der Regel ca. 13-30 Aminosäuren, die "hydrophile" Gruppe dagegen ca. 12-70 Aminosäuren. Die Signalsequenz des "hydrophoben" Typs kann in drei Strukturelemente unterteilt werden. Sie besteht aus einem relativ hydrophilen $NH_2$-Terminus mit einer oder zwei basischen Aminosäuren, einem apolaren, größtenteils hydrophoben Block aus sieben oder acht Aminosäuren sowie einem relativ hydrophilen COOH-Terminus, welcher mit einer Aminosäure mit kleiner Seitenkette endet. Solche "hydrophoben" Signalsequenzen leiten Proteine durch die Membran des endoplasmatischen Retikulums (ER) und durch Bakterienmembranen. Obwohl sich bakterielle und ER-Signalsequenzen geringfügig voneinander unterscheiden, sind sie funktionell austauschbar. Der "hydrophile" Typ unterscheidet sich in seinem Aufbau stark vom obengenannten "hydrophoben" Typ: es gibt beim "hydrophilen" Typ keine längeren nicht unterbrochenen Abschnitte hydrophober Aminosäuren, wohl aber findet man zahlreiche basische und hydroxilierte Aminosäuren und wenige oder keine sauren Aminosäuren. Der "hydrophile" Typ von Signalsequenzen leitet Proteine in Mitochondrien, Chloroplasten und möglicherweise auch in Peroxisomen. Er ist für die vorliegende Erfindung nicht von Bedeutung.

Obwohl, wie oben gezeigt, der "hydrophobe" Typ der Signalsequenzen prokaryotischer und eukaryotischer Herkunft gemeinsame Charakteristika aufweisen und funktionell austauschbar sein können, lassen sich auch Unterschiede beobachten: so weisen die meisten bisher bekannten prokaryotischen Signalsequenzen im Vergleich zum "hydrophoben" Typ (=ER Typ) eukaryotischer Signalsequenzen eine geringere Hydrophobizität im apolaren Abschnitt sowie meistens eine zusätzliche basische Aminosäure in der $NH_2$-Region auf. Möglicherweise ist das der Grund dafür, daß in der Regel die natürliche Signalsequenz eines heterologen Proteins in Mikroorganismen ineffizienter erkannt und prozessiert wird als eine diesem Protein vorgeschaltete bakterielle Signalsequenz.

Die Sekretion eines heterologen Proteins erfolgt in der Regel in E. coli als Transport durch die innere Membran in den periplasmatischen Raum; nur wenige Ausnahmen sind bekannt, in denen heterologe Proteine in das umgebende Medium sekretiert werden. Der Transport eines heterologen Proteins in den periplasmatischen Raum in E. coli entspricht funktionell weitgehend dem Transport eines Proteins in das Lumen des endoplasmatischen Retikulums von eukaryotischen Zellen. Als Folge dieses Prozesses können Proteine korrekt gefaltet und intramolekulare Disulfidbrücken auch in E. coli korrekt ausgebildet werden. Durch spezifische Signalpeptidasen wird die Signalsequenz proteolytisch abgespalten und somit das reife, "prozessierte" heterologe Protein in E. coli synthetisiert.

Manche Proteine sind nach cytoplasmatischer Expression in Bakterien, z. B. Escherichia coli, instabil und werden sehr rasch durch Proteasen wieder abgebaut. Dieser Abbau kann u. a. dadurch verhindert werden, daß diese Proteine durch eine vorgeschaltete sehr effiziente Signalsequenz rasch in den periplasmatischen Raum sezerniert werden. Es stellte sich daher die Aufgabe, besonders effiziente Signalsequenzen zu isolieren und dazu geeignete Verfahren zu konzipieren.

Hoffman und Wright (Proc.Acad.Natl.Sci. USA; (1985) 82, 5107-5111) beschreiben Plasmide, die für die periplasmatische alkalische Phosphatase aus E. coli (PhoA, EC 3.1.3.1) ohne zugehörige Signalsequenz kodieren. In in vitro Fusionen wird nun bei Fusionspartnern mit eigener Signalsequenz aktive alkalische Phosphatase in Form eines Fusionsproteins sekretiert, während bei Fehlen von einer anfusionierten Signalsequenz für die ins Cytoplasma abgegebene alkalische Phosphatase keine Aktivität nachweisbar ist. Manoil und Beckwith (Proc. Natl.Acad.Sci USA (1985) 82, 8129-8133) führten diese Arbeiten fort, indem sie die für PhoA ohne Signalsequenz und 5 anschließende Aminosäuren kodierende cDNA 3′-seitig vor das Transposon Tn5 setzten (loc.cit). und so zeigen konnten, daß nicht nur Fusionen mit sekretierten Proteinen, sondern auch mit Membranproteinen zu aktiver PhoA führen. Das genannte Konstrukt "TnPhoA" ist folglich zur Identifizierung von Signalsequenzen bzw. signalsequenzähnlichen Strukturen geeignet.

S. Knapp und J. Mekalanos (J. Bacteriology (1988) 170, 5059-5066) erzeugten nun mittels TnPhoA Mutagenese in Bordetella pertussis Mutanten, die durch Modulationssignale (hier Nicotinsäure und Mg $SO_4$) beeinflußt werden, wobei die Mehrzahl dieser Mutanten reprimiert und einige aktiviert werden, was auf mindestens zwei "trans-acting" regulatorische Gene schließen läßt.

Wir haben gefunden, daß in der dort genannten Mutante SK6 eine neue, sehr effiziente Signalsequenz enthalten ist.

Diese neue Signalsequenz gehört zu einem sekretorischen Protein aus Bordetella pertussis und besitzt die Sequenz (vgl. Tab. 2 und 3)

M K K W F V A A G I G A G L L M L S S A A.

Weiterhin werden PhoA enthaltende Plasmide beschrieben, die einerseits sehr gut als "Signalsequenz-Klonierungsvektoren" geeignet sind und andererseits einen quantitativen Vergleich verschiedener Signalsequenzen bezüglich ihrer "Sekretionseffizienz" ermöglichen. Besonders nützlich für beide Zwecke ist der Vektor pTrc99C-PhoA (Fig. 1, Tab. 1 und Beispiel 2). Dieser Vektor ist aus pTrc99C (Amann et al. Gene 69 (1988) 301-315) und einer dahingehend veränderten Signalpeptidsequenz-freien PhoA DNA so zusammengesetzt worden, daß sich das Strukturgen für PhoA im korrekten Leserahmen zum Translationsinitiationscodon von pTrc99C befindet und unmittelbar am 5′-Ende des PhoA Strukturgens (ohne Signalsequenz) eine NcoI-Schnittstelle erzeugt wurde.

Die Erfindung betrifft folglich:

a) die Signalsequenz

M K K W F V A A G I G A G L L M L S S A A

b) Plasmide, die eine solche Sequenz tragen,

c) deren Verwendung zur Sekretion von Proteinen, sowie

d) Plasmide, die besonders zur Klonierung und quantitativen Bewertung von Signalsequenzen geeignet sind, dadurch daß nach einem starken regulierbaren Promotor wie trc die lacZ Ribosomenbindungsstellle (RBS) sowie ein vektorkodiertes Translationsinitiationscodon in einem auf hohe Expression hin optimierten Abstand zur lacZ RBS folgt, wobei eine NcoI-Schnittstelle unmittelbar am 5′-Ende des signalsequenz-freien PhoA Strukturgens vorhanden ist, aber innerhalb der PhoA-Sequenz durch Mutation entfernt wurde und wobei pTrc99C-PhoA bevorzugt ist.

Die Erfindung ist ferner in den Beispielen und den Patentansprüchen weiter ausgeführt.

Beispiel 1

Identifizierung und Isolierung der Bordetella Pertussis Signalsequenz

Bei dem im folgenden verwendeten Transposon TnPhoA handelt es sich um ein Derivat des Transposons Tn5. TnPhoA trägt im linken IS50 Insertionselement ein signalsequenz-freies Derivat des E.coli PhoA Strukturgens. Dieses wurde von Manoil und Beckwith (a.a.O) derart konstruiert, daß bei erfolgter Transposition von TnPhoA in ein chromosomales oder plasmidkodiertes Gen nur dann eine PhoA-positive Genfusion resultiert, wenn die Leserahmen des E.coli PhoA Strukturgens aus TnPhoA und der Signalsequenz des durch die Transposition betroffenen Strukturgens übereinstimmen. Solche PhoA positive Kolonien können mit Hilfe des Farbstoffindikators 5-Bromo-4-Chloro-Indoxyl-Phosphat-Toluidin (XP) leicht identifiziert werden. Mit der beschriebenen Technik wurde in dem Bordetella pertussis Wildstamm 18323 eine TnPhoA Mutagenese durchgeführt (Knapp und Mekalanos (1988) a.a.O.). Dabei wurde unter anderem die PhoA-positive TnPhoA Mutante SK6 erzeugt, deren TnPhoA Genfusion als vrg-6 bezeichnet wird. Die vrg6 Genfusion wurde auf einem 20 kb BamHI Fragment im Vektorplasmid pBR322 wie folgt kloniert: Genomische DNA der Mutante SK6 wurde mit BamHI gespalten und mit BamHI geschnittener pBR322 DNA ligiert und in den E.coli Stamm CC118 (= PhoA negativ) transformiert. Klone, die das genomische Fragment mit der TnphoA Genfusion enthalten, wurden auf Kanamycin/Ampicillin Agar Platten selektioniert (TnphoA kodiert wie Tn5 für ein Kanamycinresistenzgen, welches zwischen dem 5′ phoA Teil von TnphoA und der TnphoA internen, singulären BamHI Schnittstelle lokalisiert ist).

Ein genomisches BamHI Fragment aus einer TnphoA Mutante, welches eine Kanamycinresistenz aufweist, muß deshalb auch das PhoA Strukturgen und die stromaufwärts gelegene genomische B. pertussis DNA bis zur nächsten genomischen BamHI Schnittstelle tragen. Im Falle des 20 kb großen BamHI Fragmentes, das die vrg6 Genfusion trägt, entsprechen ca. 14 kb genomischer B. pertussis DNA und ca. 6 kb der TnPhoA kodierenden DNA. Transkriptionelle und translationelle Regulationssequenzen der vrg6 Genfusion wurden weiter eingegrenzt. Hierzu wurde das 20 kb große BamHI Fragment einer Restriktionsanalyse unterzogen und Subfragmente, die die gesamte PhoA Sequenz aus TnphoA, jedoch im Vergleich zum 20 kb Fragment verkürzte B. pertussis DNA-Bereiche tragen, in pBR322 bzw. pUC18 kloniert. Die so gewonnenen Deletionsderivate wurden in das in B. pertussis replizierfähige Plasmid pLAFR2 (Friedmann et al. (1982), Gene 18, 289-196) umkloniert und nach konjugativem Transfer in B. pertussis auf modulierbare PhoA Aktivität hin untersucht. So wurde ein ca. 3,2 kb großes PstI Fragment identifiziert und in pUC18 subkloniert (im folgenden pUC-PI genannt), das nun nur noch ca. 500 Basenpaare B. pertussis DNA stromaufwärts der TnphoA Insertionsstelle der vrg6 Genfusion enthält und in B. pertusssis nach Induktion PhoA positiv ist. Da sich die PhoA Aktivität von B. pertussis Derivaten, die das klonierte 20 kb große BamHI Fragment oder das 3,2 kb große PstI Fragment enthalten, in ihrer Phosphatase Aktivität nicht wesentlich unterscheiden, müssen die transkriptionellen und translationellen Regulationssequenzen der vrg6 Genfusion auf dem letzteren Fragment noch vollständig enthalten sein. Ausgehend von pUC-PI wurde in die 500 Basenpaare stromaufwärts der TnphoA Insertionsstelle gelegenen DNA Region mit Hilfe der Enzyme Exonuklease III und S1 Nuklease nach der Methode von Henikoff ((1984) Gene 28, 351-359) Deletionen eingeführt. Dabei wurden u.a. die beiden pUC-PI Derivate vrg6-delta12 und vrg6-delta11 erhalten. vrg6-delta12 enthält noch ca. 200 Basenpaare B. pertussis spezifische DNA stromaufwärts der TnphoA Insertionsstelle und ist ebenfalls PhoA positiv. Mittels DNA Sequenzierung wurde die B. pertussis Signalsequenz auf diesem rekombinanten Plasmid bestimmt.

Die Signalsequenz lautet:

M K K W F V A A G I G A G L L M L S S A A

(vgl. auch Tab. 2). Die solchermaßen charakterisierte B. pertussis Signalsequenz umfaßt 21 Aminosäuren und wurde nachfolgend wie unter Beispiel 3 beschrieben hergestellt, kloniert und ist zur Sekretion heterologer Proteine geeignet.

vrg6-delta11 enthält lediglich vier B. pertussis spezifische Nukleotide stromaufwärts der TnphoA Insertionsstelle, gefolgt von einer pUC18 spezifizierten SacI-Schnittstelle (Tab. 1). Durch PstI/SacI Spaltung der vrg6-delta11 DNA enthält man das vollständige, signalsequenzfreie PhoA Strukturgen aus TnphoA auf einem ca. 2,6 kb großem Fragment, welches als Quelle für das signalsequenzfreie phoA-Strukturgen in Beispiel 2 dient.

## Beispiel 2

Konstruktion eines Vektorplasmides (pTrc99C-phoA) zur Klonierung und vergleichenden Effizienzmessung von Signalsequenzen

Im folgenden wird die Konstruktion des Vektorplasmides pTrc99C-phoA beschrieben. Dieses Vektorplasmid trägt als wesentliches Element das bereits oben beschriebene signalsequenzfreie phoA Strukturgen isoliert aus TnphoA. Das phoA Strukturgen trägt eine interne NcoI Schnittstelle. Diese Schnittstelle wurde mit Hilfe der Methode der zielgerichteten Mutagenese unter Beibehaltung der Aminosäuresequenz eliminiert.

Hierzu wurde zunächst das rekombinante PhoA-negative Plasmid pvrg6-delta11 (siehe Beispiel 1) mit EcoRI gespalten und das 330 Basenpaar große Fragment aus dem internen Bereich des phoA-Stukturgens isoliert. Dieses Fragment, welches die zu mutagenisierende NcoI Schnittstelle enthält, wurde in die EcoRI Stelle des Mutagenesevektors pMa5-8 (Fig.3) ligiert. Das resultierende Plasmid pMa5-8-EcoRI330 wurde isoliert und dazu benutzt, einen Einzelstrang herzustellen. Der so erhaltene Einzelstrang mit dem klonierten EcoRI Fragment wurde dann nach bekannten Methoden isoliert und dem publizierten "gapped-duplex" Mutageneseprotokoll (Kramer et al. (1984) Nucl. Acids Res. 12, 9441-9456) unterzogen, wobei folgendes Oligodesoxynukleotid verwendet wurde:

5′ ATCGATATTGCCGTGGTACGTTGCTTTC 3′

Ein Plasmid, welches die gewünschte NcoI Mutation aufwies, wurde durch entsprechende Restriktionsanalyse identifiziert, die relevante Region wurde sequenziert und als korrekt bestätigt. Aus diesem Plasmid wurde anschließend das 330 Basenpaar große EcoRI Fragment reisoliert und anstelle des entsprechenden Fragments des Plasmides pvrg-6-delta11 gesetzt. Hierzu wurde pvrg-6-delta11 partiell mit EcoRI verdaut und, verglichen mit dem durch partiellen EcoRI-Verdau linearisierten Ausgangsplasmid pvrg-delta11 (ca. 6700 bp), ein um 330 Basenpaare verkleinertes Fragment isoliert. Das EcoRI Fragment dieser Größe (ca. 6400 bp) wurde mit alkalischer Phosphatase behandelt und mit dem mutagenisierten 330 Basenpaare großen EcoRI Fragment ligiert und der Ligationsansatz in E. coli transformiert. Rekombinante Plasmide, die ein restauriertes phoA Strukturgen mit dem korrekt inserierten 330 Basenpaar EcoRI Fragment enthal ten, wurden mit Hilfe von Restriktionsanalyse und DNA Sequenzierung identifiziert. Ein solches rekombinantes Plasmid, pvrg6-delta11-deltaNcoI, wurde vermehrt und zur Konstruktion des Hybridplasmids pTrc99C-phoA verwendet. Hierzu wurde von pvrg6-delta11-deltaNcoI ein ca. 2600 Basenpaar großes SacI-ScaI Fragment isoliert. Im nächsten Schritt wurde das ca. 900 Basenpaar große SacI-ScaI Fragment aus pTrc99C (Amann et al. (1988) Gene 69, 301-315) durch dieses ca. 2600 Basenpaar große SacI-ScaI Fragment ersetzt. Das resultierende rekombinante Plasmid pTrc99C-phoA trägt als Folge obiger Manipulationen nun eine singuläre NcoI Schnittstelle unmittelbar am 5′ Ende des signalsequenzfreien phoA Strukturgens und kann, wie im folgenden Beispiel gezeigt, zur Klonierung beliebiger synthetischer oder natürlicher Signalsequenzen verwendet werden. pTrc99C-phoA trägt das Strukturgen von phoA im korrekten Leserahmen zum Translationsinitiationscodon des Expressionsvektors pTrc99C, ist aber aufgrund der Abwesenheit der phoA Signalsequenz nicht in der Lage, in transformierten Escherichia coli Zellen die Synthese einer enzymatisch aktiven alkalischen Phosphatase zu bewirken und eignet sich deshalb als "Signalsequenz-Klonierungs-Vektor". Weiterhin trägt pTrc99C-phoA stromabwärts des hybriden trc Promoters (Amann und Brosius (1985) Gene 40, 183-190) die lacZ Ribosomenbindungsstelle (RBS) sowie ein Tranlationsinitiationscodon in einem auf hohe Expression hin optimierten Abstand zur lacZ RBS. E.coli Zellen, die das rekombinante Plasmid pTrc99C-phoA enthalten, bilden keine plasmidkodierte biologisch aktive alkalische Phosphataseaktivität aus, da dem phoA Strukturgen dieses Plasmides die Signalsequenz fehlt. PhoA positive Kolonien können nun dadurch erzeugt werden, indem ein DNA Fragment kodierend für eine Signalsequenz dem phoA Strukturgen im korrekten Leserahmen vorgeschaltet wird. Dies kann dadurch geschehen, daß pTrc99C-phoA mit NcoI geschnitten wird und synthetische DNA Fragmente, die für Signalsequenzen kodieren, in diese Vektor DNA inseriert werden. Bakterienkolonien, die hybride Plasmide dieser Manipulation tragen, können nun über ihren neuen PhoA positiven Phänotyp mit Hilfe des bereits oben beschriebenen Farbstoffindikators XP leicht identifiziert werden. Das vorgestellte Prinzip wird weiter unten in Form von Ausführungsbeispielen erläutert. Werden Signalsequenzen verschiedener sekretorischer Proteine in den pTrc99C-phoA Vektor kloniert, erhält man isogene rekombinante Plasmide, die sich lediglich in der Signalsequenz unterscheiden. Aufgrund dessen gibt die phoA Aktivität der E. coli Zellen, die solche Konstrukte enthalten, ein Maß für die Effizienz der betreffenden klonierten Signalsequenzen.

Eine weitere Verwendungsmöglichkeit für den Vektor pTrc99C-phoA besteht darin, synthetische DNA Fragmente zu klonieren, die nicht für eine eindeutig definierte Signalsequenz codieren, sondern dergestalt degeneriert sind, daß für jede Position der Signalsequenz eine Vielzahl möglicher Aminosäuren in Frage kommt. Es handelt sich hierbei in gewisser Weise um eine "shotgun" Klonierung, wobei die vektorbedingt nun mögliche phoA-Aktivitätsmessung ein Maß für die Effizienz der artifiziellen Signalsequenz darstellt. Mit dieser Methode lassen sich neue, für die heterologe Expression klonierter Gene benutzbare Signalsequenzen herstellen und bewerten.

Das Konstruktionsprinzip von pTrc99C-phoA ist in Fig. 1 verdeutlicht. Die Abkürzungen bedeuten: N = NcoI, S = SacI, P = PstI, [N] = NcoI Stelle wird nach Ligation nicht regeneriert, 'phoA = signalsequenzloses phoA Strukturgen, Pfeile geben die Transkriptionsrichtung bzw. die $NH_2 \rightarrow COOH$ Orientierung translatierter Regionen an. Oligo bedeutet = synthetische Oligonukleotidsequenz. Die Tab. 1 zeigt die relevante Klonierungs- und Translationinitiationsregion von pTrc99C-phoA.

## Beispiel 3

DNA Synthese und Klonierung der Bordetella pertussis Signalsequenz sowie fünf weiterer natürlich vorkommender mikrobieller Signalsequenzen sekretorischer Proteine

Mit Hilfe des Vektors pTrc99C-phoA wurden sechs verschiedene Signalsequenzen kloniert, deren Aminosäuresequenzen in der Tab. 2 dargestellt sind. Neben der neuen Bordetella pertussis Signalsequenz wurden fünf weitere Signalsequenzen nach folgenden Kriterien ausgewählt:

a) Signalsequenz eines periplasmatischen Proteins

- Alkalische Phosphatase (phoA) aus E. coli (Kikuchi et al. (1981) Nucleic Acids Res. 9, 5671-5678)

b) Signalsequenz eines äußeren Membranproteins

- Outer membrane protein (ompA) aus E. coli (Movva et al. (1980) J.Biol.Chem. 255, 27-29

c) Signalsequenzen dreier ins Medium sezernierter Proteine

- Heat stable toxin I (STI) aus E. coli (So und McCarthy (1980) Proc.Natl.Acad.Sci. USA 77, 4011-4015)
- Heat stable toxin II (STII) aus E. coli (Lee et al. (1983) Infect.Immun. 42, 264-268)
- Amylase aus Bacillus subtilis (Yang et al. (1983) Nucleic Acids Res. 11, 237-249)

Für die Synthese und Klonierung dieser Signalsequenzen wurde folgende vereinfachte Nomenklatur verwendet:

Bordetella pertussis vrg-6 Signalsequenz = Seq 1
PhoA Signalsequenz = Seq 2
OmpA Signalsequenz = Seq 3
STI Signalsequenz = Seq 4
STII Signalsequenz = Seq 5
Bacillus subtilis Amylase Signalsequenz = Seq 6

Alle sechs genannten Signalsequenzen wurden mittels DNA Synthese hergestellt. Die dazu synthetisierten DNA Fragmente (in der Tab. 3 dargestellt) wurden unter Verwendung der unter Beispiel 2 beschriebenen Selektion auf Alkalische Phosphatase im Testvektor pTrc99C-phoA kloniert und identifiziert. Die synthetischen Signalsequenz-kodierenden DNA Fragmente wurden dergestalt konzipiert, daß nach Insertion in der korrekten Orientierung im Vektor pTrc99C-phoA nur eine NcoI Stelle regeneriert wird, und zwar stromabwärts der Signalsequenz kodierenden Region (vgl. auch die Fig. 1, Tab. 3 und Tab. 4). Somit kann diese NcoI Stelle weiterhin, wie unter Beispiel 4 weiter ausgeführt, als Klonierungsstelle für die Insertion heterologer Gene in die pSEC Vektoren benutzt werden (pSEC = Sekretion).

Die Synthese der in Tab. 3 gezeigten zwölf DNA Fragmente erfolgte nach bekannten Methoden (Sinha et al. (1984) Nucl. Acids Res. 12, 4539-4557) unter Verwendung von ß-Cyanoethylamiditen. Die Synthesen wurden mit einem Synthesizer der Firma Biosearch nach der Phosphit-Triester-Methode (Letsinger (1975) J.Amer.Chem.Soc. 97, 3278; Letsinger (1976) J.Amer.Chem.Soc. 98, 3655) durchgeführt. Nach der Abspaltung vom Träger (CPG) mit konz. Ammoniak bei Raumtemperatur über 5-8 h und der Abspaltung der Schutzgruppen an den Basen in der gleichen Lösung bei 55° C über etwa 12 h wurden die Oligodesoxynukleotide gelelektrophoretisch bzw. durch "reverse-phase" HPLC gereinigt. Die Oligodesoxynukleotide wurden in Annealingpuffer (100 mM NaCl, 10 mM TRIS-Cl (pH 7,8), 0,1 mM EDTA) aufgenommen, jeweils molare Mengen der beiden Stränge zusammengegeben, für 5 min bei 95° C inkubiert und langsam auf Raumtemperatur abgekühlt. Die doppelsträngigen DNA Fragmente weisen an den 5′ Enden vier Basen lange Einzelstrangregionen komplementär zu einer NcoI-Erkennungsstelle auf. Der Testvektor pTrc99C-phoA wurde mit NcoI linearisiert und in verschiedenen Ansätzen zusammen mit hybridisierten DNA-Fragmenten ligiert. Kompetente E. coli Zellen wurden nach bekannten Methoden mit den Ligationsansätzen transformiert, auf LB/Amp Agarplatten ausplattiert und bei 37° C über Nacht inkubiert. Die Kolonien wurden mittels der Methode der Replica-Plattierung auf LB/Amp/XP/IPTG Indikatorplatten überimpft und erneut bei 37° C inkubiert. PhoA positive Kolonien sind auf dieser Indikatorplatte blau gefärbt. Plasmid DNA dieser Kolonien wurde isoliert und sequenziert, wobei die korrekte Orientierung der synthetischen DNA-Fragmente sowie die zu erwartende korrekte Signalsequenz für die sechs obengenannten Beispiele bestätigt werden konnte. Die so erhaltenen Plasmide mit der durch Sequenzierung als korrekt bestätigten jeweiligen Signalsequenz wurden entsprechend obiger Tabelle als pTrc99C-phoA-Seq-1, -2, -3, -4, -5 und -6 bezeichnet. Unter standardisierten Bedingungen lassen sich nun anhand der Extinktion (Messung des freigesetzten Farbstoffs) diese Signalsequenzen vergleichen und bewerten, wobei die gefundene aus B. pertussis zu den relativ stärksten gehört.

## Beispiel 4

Konstruktion der Sekretions-Vektoren pSEC-Bp-1, pSEC-Bp-2 und pSEC-Bp-3

Plasmid DNA des Klones pTrc99C-phoA-Seq-1 wurde mit SacI und ScaI verdaut und das ca. 3,1 kb große Fragment isoliert. Dieses Fragment trägt zusätzlich zu der B. pertussis Signalsequenz ausschließlich pTrc99C spezifische Sequenzen (siehe auch Fig. 1). Dieses Fragment wurde in drei getrennten Ansätzen jeweils mit einem der ca. 0,9 kb SacI/ScaI Fragmente der Plasmide pTrc97A, pTrc97B und pTrc97C (Amann et al. a.a.O.) ligiert und die resultierenden Plasmide als pSEC-Bp-1, pSEC-Bp-2 und pSEC-Bp-3 bezeichnet. Durch diese Manipulation wurde die lange Polylinkerregion der Plasmide pTrc97A, pTrc97B und pTrc97C genutzt, um in allen drei Leserahmen eine Vielzahl von Restriktionsstellen stromabwärts von der Bordetella pertussis Signalsequenz-codierenden Region zur Verfügung zu stellen (Tab. 4). Analog zu diesen Konstruktionen können ähnliche Sekretionsvektoren zur Expression und Sekretion heterologer Proteine unter Verwendung der Plasmide pTrc99C-phoA-Seq2, -3, -4, -5 und -6 hergestellt werden. Die so hergestellten Sekretionsvektoren unterscheiden sich in ihrer relativen Effizienz sowie in der zellulären Lokalisation des exprimierten Produktes entsprechend der Herkunft der jeweils verwendeten Signalsequenz.

Fig. 2 zeigt beispielhaft die Plasmidstruktur von pSEC-BP1 und Tab. 5 die vollständige DNA-Sequenz von pSEC-BP1, wobei xxx für ein Start- bzw. Stop-Codon steht.

Legende zu Fig. 1:

Karte der Plasmide pMAC5-8 (= pMA5-8 und pMC5-8).
F1-ORI: Replikationsursprung des Phagen f1;
ORI: Replikationsursprung vom Co1E1-Typ;
CAT: Kodierende Region für Chloramphenicol-acetyl-transferase;
AMP: Kodierende Region für ß-Lactamase.
pMA5-8 trägt eine amber-Mutation in CAT (A bei Position 3409) und pMC5-8 eine amber-Mutation in AMP (C bei Position 2238).

pTrc99C-phoA

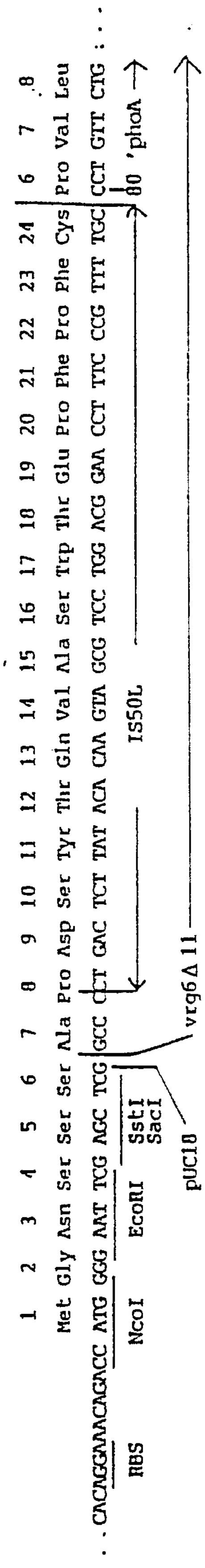


Tab. 1

| Sequenz (-30 … -20 … -10 … -1) | | Aminosäuren |
|---|---|---|
| M K K W F V A A G I G A G L L M L S S A A | B.p. | 21 |
| M K Q S T I A L A L L P L L F T P V T K A | E.c. phoA | 21 |
| M K K T A I A I A V A L A G F A T V A Q A | E.c. ompA | 21 |
| M K K L M L A I F F S V L S F P S F S Q S | E.c. ST I | 21 |
| M K K N I A F L L A S M F V F S I A T N A Y A | E.c. ST II | 23 |
| M F A K R F K T S L L P L F A G F L L L F H L V L A G P A A A S | B.s. Amylase | 32 |

Tab. 2

Tab. 3

Bordetella pertussis Signalsequenz:

```
5' C ATG AAA AAG TGG TTC GTT GCT GCC GGC ATC GGC GCT GCC GGA CTC ATG CTC TCC AGC GCC GC
         TTT TTC ACC AAG CAA CGA CGG CCG TAG CCG CGA CGG CCT GAG TAC GAG AGG TCG CGG CGG TAC 5'
```

E. coli phoA Signalsequenz:

```
5' C ATG AAA CAA AGC ACT ATT GCA CTG GCA CTC TTA CCG TTA CTG TTT ACC CCT GTG ACA AAA GC
         TTT GTT TCG TGA TAA CGT GAC CGT GAG AAT GGC AAT GAC AAA TGG GGA CAC TGT TTT CGG TAC 5'
```

E. coli ompA Signalsequenz:

```
5' C ATG AAA AAG ACA GCT ATC GCG ATT GCA GTG GCA CTG GCT GGT TTC GCT ACC GTA GCG CAG GC
         TTT TTC TGT CGA TAG CGC TAA CGT CAC CGT GAC CGA CCA AAG CGA TGG CAT CGC GTC CGG TAC 5'
```

E. coli heat-stable toxin I Signalsequenz:

```
5' C ATG AAA AAG CTA ATG TTG GCA ATT TTT ATT TCT GTA TTA TCT TTC CCC TCT TTT AGT CAG TCA CC
         TTT TTC GAT TAC AAC CGT TAA AAA TAA AGA CAT AAT AGA AAG GGG AGA AAA TCA GTC AGT GGG TAC 5'
```

E. coli heat-stable toxin II Signalsequenz:

```
5' C ATG AAA AAG AAT ATC GCA TTT CTT CTT GCA TCT ATG TTC GTT TTT TCT ATT GCT ACA AAT GCC TAT GC
         TTT TTC TTA TAG CGT AAA GCC GAA CGT AGA TAC AAG CAA AAA AGA TAA CGA TGT TTA CGG ATA CGG TAC 5'
```

Bacillus subtilis Amylase Signalsequenz:

```
5' C ATG TTT GCA AAA CGA TTC AAA ACC TCT TTA CTG CCG TTA TTC GCT GGA TTT TTA TTG CTG TTT CAT TTG GTT
         AAA CGT TTT GCT AAG TTT TGG AGA AAT GAC GGC AAT AAG CGA CCT AAA AAT AAC GAC AAA GTA AAC CAA

   CTG GCA GGA CCG GCG GCT GCG AGT CC
   GAC CGT CCT GGC CGC CGA CGC TCA GGG TAC 5'
```

Tab. 4

**pSEC-Bp1**

B. p. Signalpeptid

```
17  18  19  20  21  | 1   2   3   4   5   6   7   8   9   10  11  12  13  14  15  16  17  18  19  20
Leu Ser Ser Ala Ala | Met Glu Phe Glu Leu Gly Thr Arg Gly Ser Ser Arg Val Asp Leu Gln Ala Cys Lys Leu  rrnB
CTC TCC AGC GCC GCC   ATG GAA TTC GAG CTC GGT ACC CGG GGA TCC TCT AGA GTC GAC CTG CAG GCA TGC AAG CTT GGCTG
                      NcoI    EcoRI   SstI    KpnI    SmaI BamHI  XbaI    SalI    PstI    SphI    HindIII
                                                      XmaI                AccI
                                                                          HincII
```

**pSEC-Bp2**

B. p. Signalpeptid

```
17  18  19  20  21  | 1   2   3   4   5   6   7   8   9   10  11
Leu Ser Ser Ala Ala | Met Gly Ile Arg Ala Arg Tyr Pro Gly Ile Leu                                rrnB
CTC TCC AGC GCC GCC   ATG GGA ATT CGA GCT CGG TAC CCG GGG ATC CTC TAG AGTCGACCTGCAGGCATGCAAGCTTGGCTG
                      NcoI    EcoRI   SstI    KpnI    SmaI    BamHI   XbaI    SalI PstI SphI HindIII
                                                      XmaI                    AccI
                                                                              HincII
```

**pSEC-Bp3**

B. p. Signalpeptid

```
17  18  19  20  21  | 1   2   3   4   5   6   7   8   9   10  11  12  13  14  15  16  17  18  19  20  21
Leu Ser Ser Ala Ala | Met Gly Asn Ser Ser Val Val Pro Gly Asp Pro Leu Glu Ser Thr Cys Arg His Ala Ser Leu  rrnB
CTC TCC AGC GCC GCC   ATG GGG AAT TCG AGC TCG GTA CCC GGG GAT CCT CTA GAG TCG ACC TGC AGG CAT GCA AGC TTG GCTG
                      NcoI    EcoRI   SstI    KpnI    SmaI BamHI  XbaI    SalI    PstI    SphI    HindIII
                                                      XmaI                AccI
                                                                          HincII
```

Tab. 5

```
   1  GTTTGACAGC TTATCATCGA CTGCACGGTG CACCAATGCT TCTGGCGTCA

  51  GGCAGCCATC GGAAGCTGTG GTATGGCTGT GCAGGTCGTA AATCACTGCA

 101  TAATTCGTGT CGCTCAAGGC GCACTCCCGT TCTGGATAAT GTTTTTTGCG
                                                     -35
 151  CCGACATCAT AACGGTTCTG GCAAATATTC TGAAATGAGC TGTTGACAAT
          trcP        -10
 201  TAATCATCCG GCTCGTATAA TGTGTGGAAT TGTGAGCGGA TAACAATTTC

                          M  K  K  W  F  V  A  A  G  I  G
 251  ACACAGGAAA CAGACCATGA AAAAGTGGTT CGTTGCTGCC GGCATCGGCG
                        ***

      A  G  L  L  M  L  S  S  A  A
 301  CTGCCGGACT CATGCTCTCC AGCGCCGCCA TGGAATTCGA GCTCGGTACC
                                       -------------------------
                                        NcoI  EcoRI  SstI  KpnI

 351  CGGGGATCCT CTAGAGTCGA CCTGCAGGCA TGCAAGCTTG GCTGTTTTGG
      ------------------------------------------
      SmaI BamHI XbaI   SalI  PstI  SphI HindIII

 401  CGGATGAGAG AAGATTTTCA GCCTGATACA GATTAAATCA GAACGCAGAA
          ***                  ***         ***

 451  GCGGTCTGAT AAAACAGAAT TTGCCTGGCG GCAGTAGCGC GGTGGTCCCA

 501  CCTGACCCCA TGCCGAACTC AGAAGTGAAA CGCCGTAGCG CCGATGGTAG

 551  TGTGGGGTCT CCCCATGCGA GAGTAGGGAA CTGCCAGGCA TCAAATAAAA

 601  CGAAAGGCTC AGTCGAAAGA CTGGGCCTTT CGTTTTATCT GTTGTTTGTC

 651  GGTGAACGCT CTCCTGAGTA GGACAAATCC GCCGGGAGCG GATTTGAACG

 701  TTGCGAAGCA ACGGCCCGGA GGGTGGCGGG CAGGACGCCC GCCATAAACT

 751  GCCAGGCATC AAATTAAGCA GAAGGCCATC CTGACGGATG GCCTTTTTGC

 801  GTTTCTACAA ACTCTTTTTG TTTATTTTTC TAAATACATT CAAATATGTA

 851  TCCGCTCATG AGACAATAAC CCTGATAAAT GCTTCAATAA TATTGAAAAA

 901  GGAAGAGTAT GAGTATTCAA CATTTCCGTG TCGCCCTTAT TCCCTTTTTT

 951  GCGGCATTTT GCCTTCCTGT TTTTGCTCAC CCAGAAACGC TGGTGAAAGT

1001  AAAAGATGCT GAAGATCAGT TGGGTGCACG AGTGGGTTAC ATCGAACTGG

1051  ATCTCAACAG CGGTAAGATC CTTGAGAGTT TTCGCCCCGA AGAACGTTTT

1101  CCAATGATGA GCACTTTTAA AGTTCTGCTA TGTGGCGCGG TATTATCCCG

1151  TGTTGACGCC GGGCAAGAGC AACTCGGTCG CCGCATACAC TATTCTCAGA
```

```
1201  ATGACTTGGT TGAGTACTCA CCAGTCACAG AAAAGCATCT TACGGATGGC
1251  ATGACAGTAA GAGAATTATG CAGTGCTGCC ATAACCATGA GTGATAACAC
1301  TGCGGCCAAC TTACTTCTGA CAACGATCGG AGGACCGAAG GAGCTAACCG
1351  CTTTTTTGCA CAACATGGGG GATCATGTAA CTCGCCTTGA TCGTTGGGAA
1401  CCGGAGCTGA ATGAAGCCAT ACCAAACGAC GAGCGTGACA CCACGATGCC
1451  TACAGCAATG GCAACAACGT TGCGCAAACT ATTAACTGGC GAACTACTTA
1501  CTCTAGCTTC CCGGCAACAA TTAATAGACT GGATGGAGGC GGATAAAGTT
1551  GCAGGACCAC TTCTGCGCTC GGCCCTTCCG GCTGGCTGGT TTATTGCTGA
1601  TAAATCTGGA GCCGGTGAGC GTGGGTCTCG CGGTATCATT GCAGCACTGG
1651  GGCCAGATGG TAAGCCCTCC CGTATCGTAG TTATCTACAC GACGGGGAGT
1701  CAGGCAACTA TGGATGAACG AAATAGACAG ATCGCTGAGA TAGGTGCCTC
1751  ACTGATTAAG CATTGGTAAC TGTCAGACCA AGTTTACTCA TATATACTTT
1801  AGATTGATTT AAAACTTCAT TTTTAATTTA AAAGGATCTA GGTGAAGATC
1851  CTTTTTGATA ATCTCATGAC CAAAATCCCT TAACGTGAGT TTTCGTTCCA
1901  CTGAGCGTCA GACCCCGTAG AAAAGATCAA AGGATCTTCT TGAGATCCTT
1951  TTTTTCTGCG CGTAATCTGC TGCTTGCAAA CAAAAAAACC ACCGCTACCA
2001  GCGGTGGTTT GTTTGCCGGA TCAAGAGCTA CCAACTCTTT TTCCGAAGGT
2051  AACTGGCTTC AGCAGAGCGC AGATACCAAA TACTGTCCTT CTAGTGTAGC
2101  CGTAGTTAGG CCACCACTTC AAGAACTCTG TAGCACCGCC TACATACCTC
2151  GCTCTGCTAA TCCTGTTACC AGTGGCTGCT GCCAGTGGCG ATAAGTCGTG
2201  TCTTACCGGG TTGGACTCAA GACGATAGTT ACCGGATAAG GCGCAGCGGT
2251  CGGGCTGAAC GGGGGGTTCG TGCACACAGC CCAGCTTGGA GCGAACGACC
2301  TACACCGAAC TGAGATACCT ACAGCGTGAG CTATGAGAAA GCGCCACGCT
2351  TCCCGAAGGG AGAAAGGCGG ACAGGTATCC GGTAAGCGGC AGGGTCGGAA
2401  CAGGAGAGCG CACGAGGGAG CTTCCAGGGG GAAACGCCTG GTATCTTTAT
2451  AGTCCTGTCG GGTTTCGCCA CCTCTGACTT GAGCGTCGAT TTTTGTGATG
2501  CTCGTCAGGG GGGCGGAGCC TATGGAAAAA CGCCAGCAAC GCGGCCTTTT
2551  TACGGTTCCT GGCCTTTTGC TGGCCTTTTG CTCACATGTT CTTTCCTGCG
```

2601 TTATCCCCTG ATTCTGTGGA TAACCGTATT ACCGCCTTTG AGTGAGCTGA
2651 TACCGCTCGC CGCAGCCGAA CGACCGAGCG CAGCGAGTCA GTGAGCGAGG
2701 AAGCGGAAGA GCGCCTGATG CGGTATTTTC TCCTTACGCA TCTGTGCGGT
2751 ATTTCACACC GCATATGGTG CACTCTCAGT ACAATCTGCT CTGATGCCGC
2801 ATAGTTAAGC CAGTATACAC TCCGCTATCG CTACGTGACT GGGTCATGGC
2851 TGCGCCCCGA CACCCGCCAA CACCCGCTGA CGCGCCCTGA CGGGCTTGTC
2901 TGCTCCCGGC ATCCGCTTAC AGACAAGCTG TGACCGTCTC CGGGAGCTGC
2951 ATGTGTCAGA GGTTTTCACC GTCATCACCG AAACGCGCGA GGCAGCAGAT
3001 CAATTCGCGC GCGAAGGCGA AGCGGCATGC ATTTACGTTG ACACCATCGA
3051 ATGGTGCAAA ACCTTTCGCG GTATGGCATG ATAGCGCCCG GAAGAGAGTC
3101 AATTCAGGGT GGTGAATGTG AAACCAGTAA CGTTATACGA TGTCGCAGAG
3151 TATGCCGGTG TCTCTTATCA GACCGTTTCC CGCGTGGTGA ACCAGGCCAG
3201 CCACGTTTCT GCGAAAACGC GGGAAAAAGT GGAAGCGGCG ATGGCGGAGC
3251 TGAATTACAT TCCCAACCGC GTGGCACAAC AACTGGCGGG CAAACAGTCG
3301 TTGCTGATTG GCGTTGCCAC CTCCAGTCTG GCCCTGCACG CGCCGTCGCA
3351 AATTGTCGCG GCGATTAAAT CTCGCGCCGA TCAACTGGGT GCCAGCGTGG
3401 TGGTGTCGAT GGTAGAACGA AGCGGCGTCG AAGCCTGTAA AGCGGCGGTG
3451 CACAATCTTC TCGCGCAACG CGTCAGTGGG CTGATCATTA ACTATCCGCT
3501 GGATGACCAG GATGCCATTG CTGTGGAAGC TGCCTGCACT AATGTTCCGG
3551 CGTTATTTCT TGATGTCTCT GACCAGACAC CCATCAACAG TATTATTTTC
3601 TCCCATGAAG ACGGTACGCG ACTGGGCGTG GAGCATCTGG TCGCATTGGG
3651 TCACCAGCAA ATCGCGCTGT TAGCGGGCCC ATTAAGTTCT GTCTCGGCGC
3701 GTCTGCGTCT GGCTGGCTGG CATAAATATC TCACTCGCAA TCAAATTCAG
3751 CCGATAGCGG AACGGGAAGG CGACTGGAGT GCCATGTCCG GTTTTCAACA
3801 AACCATGCAA ATGCTGAATG AGGGCATCGT TCCCACTGCG ATGCTGGTTG
3851 CCAACGATCA GATGGCGCTG GGCGCAATGC GCGCCATTAC CGAGTCCGGG
3901 CTGCGCGTTG GTGCGGATAT CTCGGTAGTG GGATACGACG ATACCGAAGA
3951 CAGCTCATGT TATATCCCGC CGTCAACCAC CATCAAACAG GATTTTCGCC

```
4001  TGCTGGGGCA AACCAGCGTG GACCGCTTGC TGCAACTCTC TCAGGGCCAG
4051  GCGGTGAAGG GCAATCAGCT GTTGCCCGTC TCACTGGTGA AAAGAAAAAC
4101  CACCCTGGCG CCCAATACGC AAACCGCCTC TCCCCGCGCG TTGGCCGATT
4151  CATTAATGCA GCTGGCACGA CAGGTTTCCC GACTGGAAAG CGGGCAGTGA
4201  GCGCAACGCA ATTAATGTGA GTTAGCGCGA ATTGATCTG
```

## Ansprüche

1. Signalpeptid aus Bordetella pertussis mit der Aminosäuresequenz
M K K W F V A A G I G A G L L M L S S A A.

2. DNA oder RNA kodierend für das Signalpeptid nach Anspruch 1.

3. Vektoren, Plasmide, DNA- oder RNA-Konstrukte, die eine DNA- oder RNA-Sequenz nach Anspruch 2 enthalten.

4. Verfahren zur Sekretion von Proteinen in gramnegativen Bakterien, dadurch gekennzeichnet, daß eine DNA oder RNA nach Anspruch 2 vor das betreffende Strukturgen positioniert wird.

5. Verwendung eines Signalpeptids nach Anspruch 1 zur Sekretion von Proteinen in gramnegativen Bakterien.

6. Expressionsvektoren, die aufeinanderfolgend
(a) einen starken, regulierbaren Promotor, vorzugsweise trc,
(b) die lacZ Ribosomenbindungsstelle und
(c) das signalsequenzfreie Strukturgen der periplasmatischen alkalischen Phosphatase (phoA) im richtigen Leserahmen zum Vektor ATG-Start, wobei die NcoI-Spaltstelle innerhalb von phoA entfernt ist und eine solche unmittelbar am 5′-Ende des signalsequenzfreien phoA-Strukturgens vorhanden ist,
besitzen.

7. Plasmid pTrc99C-phoA.

8. Verfahren zur Isolierung von Signalsequenzen, dadurch gekennzeichnet, daß Expressionsvektoren nach Anspruch 6 eingesetzt werden.

9. Verfahren zur quantitativen Bewertung von Signalsequenzen, dadurch gekennzeichnet, daß Expressionsvektoren nach Anspruch 6 eingesetzt werden.

10. Verfahren nach Anspruch 8 oder 9, dadurch gekennzeichnet, daß pTrc99C-phoA eingesetzt wird.

11. Verwendung von Expressionsvektoren nach Anspruch 6 oder 7 zur quantitativen Bewertung von Signalsequenzen.

Patentansprüche für folgende Vertragstaaten: GR, ES

1. Verfahren zur Sekretion von Proteinen in gramnegativen Bakterien, dadurch gekennzeichnet, daß ein Signalpeptid aus Bordetella pertussis mit der Aminosäuresequenz
M K K W F V A A G I G A G L L M L S S A A
vor das betreffenden Strukturgen positioniert wird.

2. Verfahren zur Isolierung von Signalsequenzen, dadurch gekennzeichnet, daß Expressionsvektoren eingesetzt werden, die aufeinanderfolgend
(a) einen starken, regulierbaren Promotor, vorzugsweise trc,
(b) die lacZ Ribosomenbindungsstelle und
(c) das signalsequenzfreie Strukturgen der periplasmatischen alkalischen Phosphatase (phoA) im richtigen Leserahmen zum Vektor, wobei die NcoI-Spaltstelle innerhalb von phoA entfernt ist und eine solche unmittelbar am 5′-Ende des signalsequenzfreien phoA-Strukturgens vorhanden ist,
besitzen.

3. Verfahren nach Anspruch 2, dadurch gekennzeichnet, daß das Plasmid pTrc99C - phoA eingesetzt wird.

4. Verfahren zur quantitativen Bewertung von Signalsequenzen, dadurch gekennzeichnet, daß Expressionsvektoren eingesetzt werden, die aufeinanderfolgend
(a) einen starken, regulierbaren Promotor, vorzugsweise trc,
(b) die lacZ Ribosomenbindungsstelle und
(c) das signalsequenzfreie Strukturgen der periplasmatischen alkalischen Phosphatase (phoA) im richtigen Leserahmen zum Vektor, wobei die NcoI-Spaltstelle innerhalb von phoA entfernt ist und eine solche unmittelbar am 5′-Ende des signalsequenzfreien phoA-Strukturgens vorhanden ist,
besitzen.

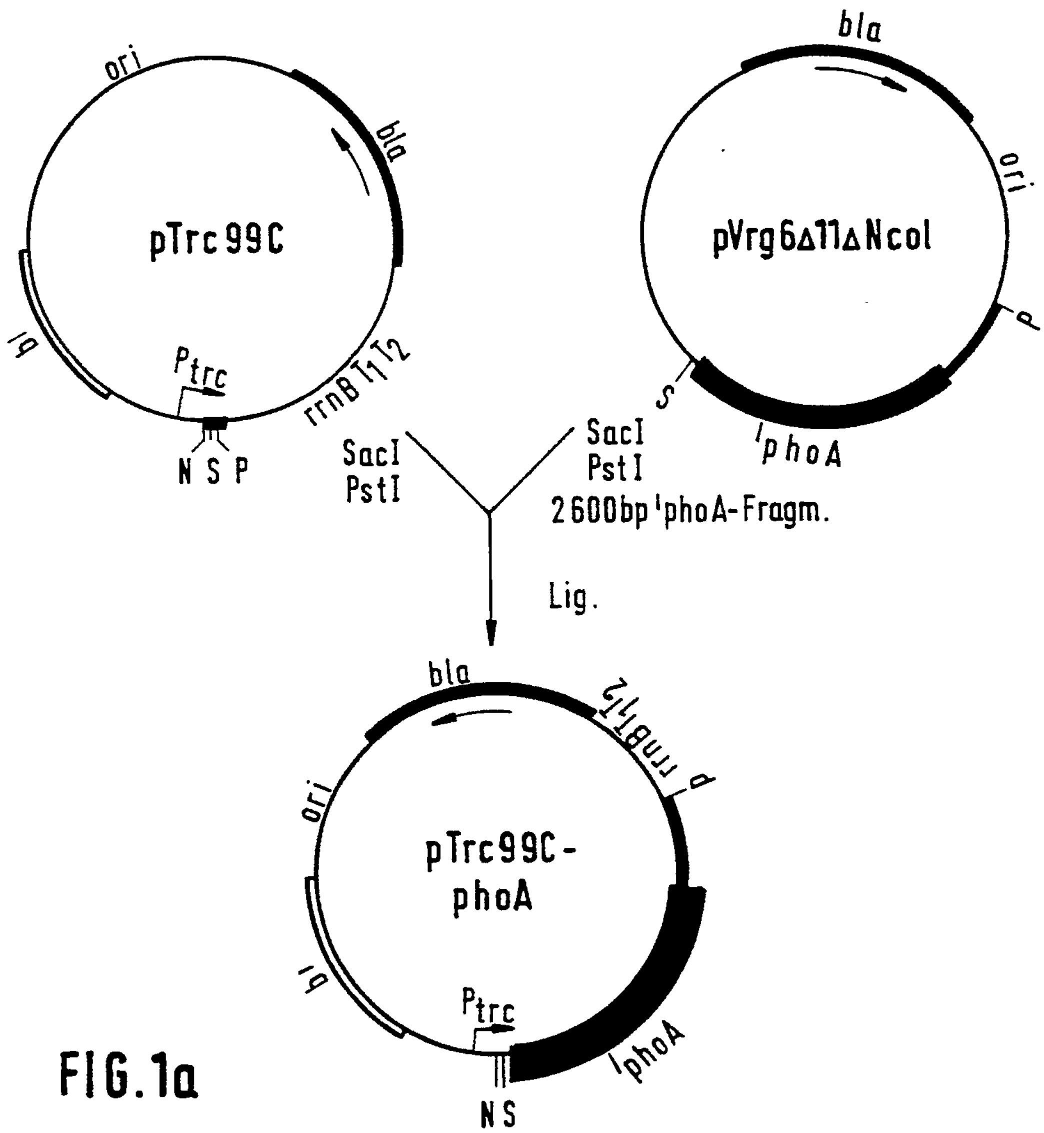
ori
bla
pTrc99C
lpl
Ptrc
rrnBT1T2
N S P
bla
ori
pVrg6Δ11ΔNcol
p
s
'phoA
SacI
PstI
SacI
PstI
2600bp 'phoA-Fragm.
Lig.
bla
rrnBT1T2
p
ori
pTrc99C-
phoA
lpl
Ptrc
'phoA
NS

FIG.1a

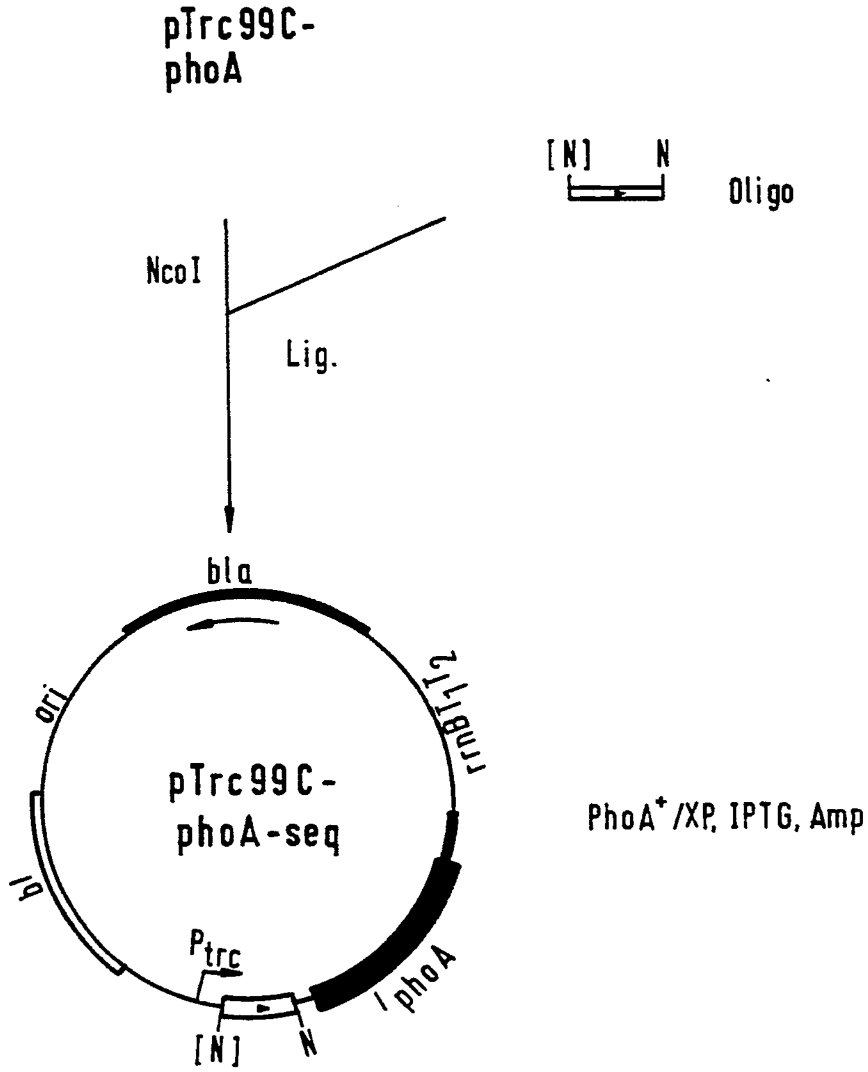
pTrc99C-
phoA
[N]
N
Oligo
NcoI
Lig.
bla
ori
rrnBT1T2
pTrc99C-
phoA-seq
lacIq
Ptrc
[N]
N
phoA
PhoA+/XP, IPTG, Amp

FIG. 1b

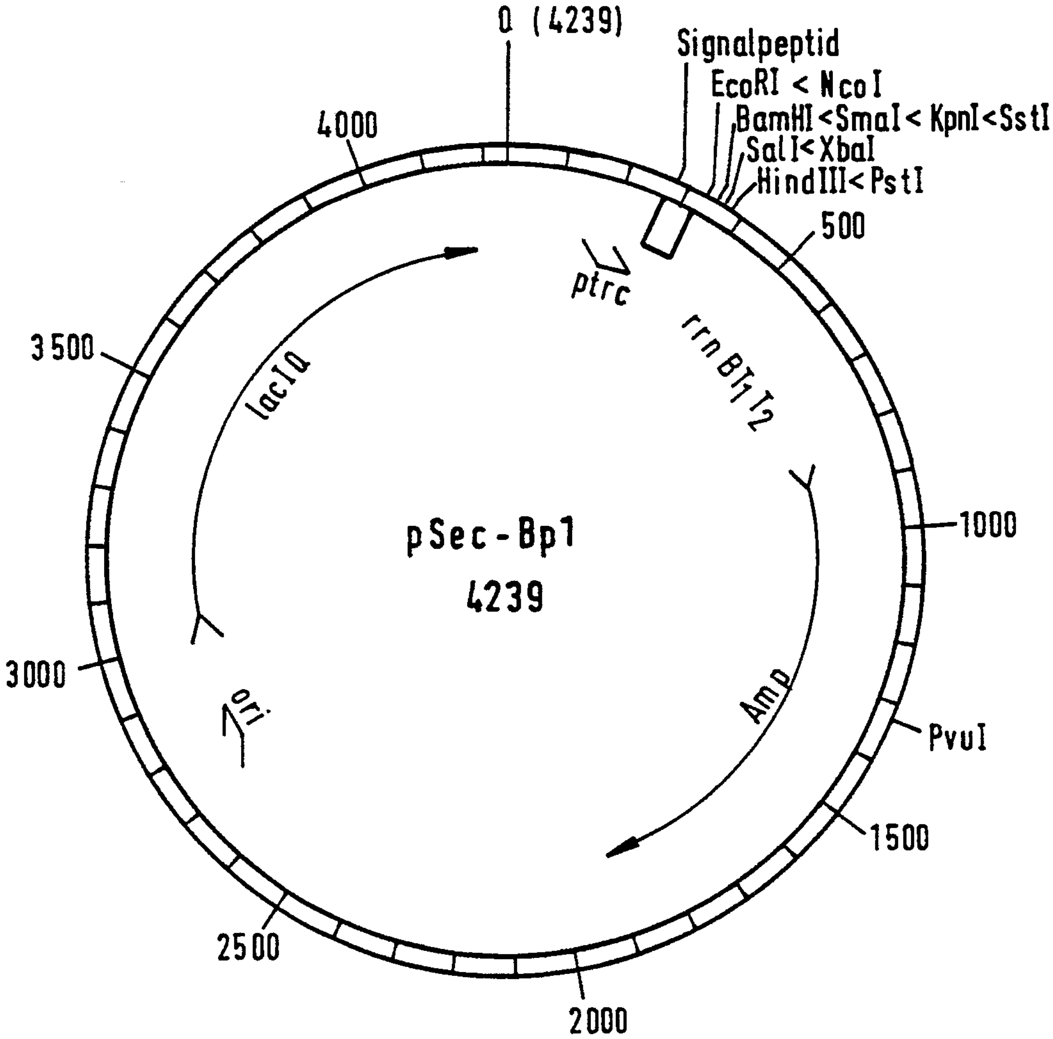
0 (4239)
Signalpeptid
EcoRI < NcoI
BamHI<SmaI< KpnI<SstI
SalI<XbaI
HindIII<PstI
500
1000
PvuI
1500
2000
2500
3000
3500
4000
ptrc
rrnBT1T2
lacIQ
ori
Amp
pSec-Bp1
4239

FIG. 2

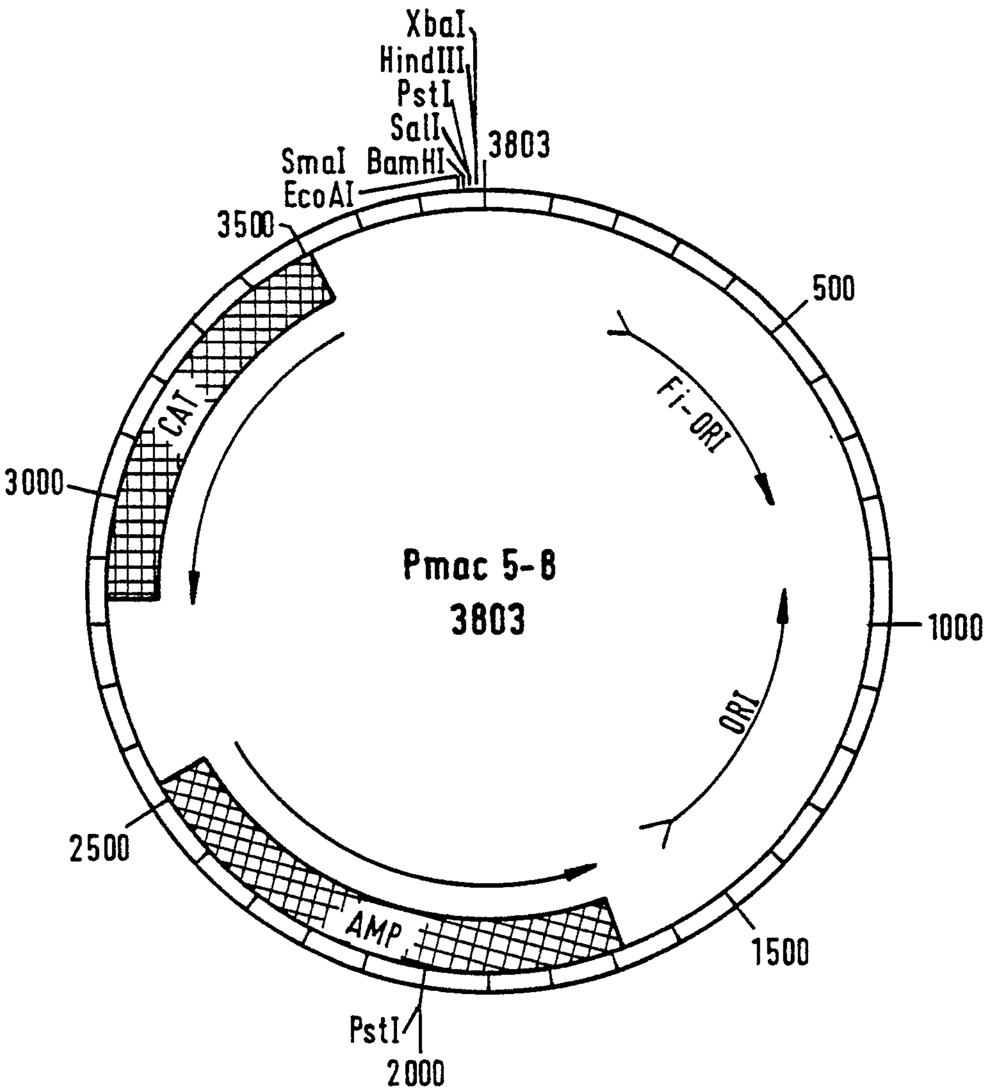

XbaI
HindIII
PstI
SalI
SmaI
BamHI
EcoAI
3803
3500
500
CAT
Fi-ORI
3000
Pmac 5-8
3803
1000
ORI
2500
AMP
1500
PstI
2000


FIG. 3